(19)

Europäisches
Patentamt
European
Patent Office
Office européen
des brevets

(11) **EP 2 165 642 A1**

(12) **EUROPEAN PATENT APPLICATION**
published in accordance with Art. 153(4) EPC

(43) Date of publication:
**24.03.2010 Bulletin 2010/12**

(21) Application number: **08765207.9**

(22) Date of filing: **05.06.2008**

(51) Int Cl.:
*A61B 1/04* (2006.01)          *H04N 5/225* (2006.01)

(86) International application number:
**PCT/JP2008/060394**

(87) International publication number:
**WO 2008/149952 (11.12.2008 Gazette 2008/50)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **08.06.2007   JP 2007152952**

(71) Applicant: **Olympus Corporation
Tokyo 151-0072 (JP)**

(72) Inventor: **SUZUKI, Hiroshi
Tokyo 151-0072 (JP)**

(74) Representative: **Schmidt, Steffen
Wuesthoff & Wuesthoff
Patentanwälte
Schweigerstrasse 2
81541 München (DE)**

(54) **IMAGE PROCESSING DEVICE, SCOPE, AND ENDOSCOPE HAVING THEM**

(57)     An object is to provide an image processor that realizes a color conversion process appropriate for an image acquisition unit such as a scope and that can improve the accuracy of color reproduction. An image processor (1) is employed that includes a plurality of color conversion sections (115); an information acquisition section (250) that acquires an index for selecting at least one of the plurality of color conversion sections (115) as a specific color conversion means for applying a color conversion process to an input video signal; and a selection section (251) that selects the at least one of the plurality of color conversion sections (115) as the specific color conversion means based on the index, in which the plurality of color conversion sections (115) include a linear color conversion section (202) that applies a color conversion process based on linear conversion to the input video signal and a table conversion section (203) that applies a color conversion process based on nonlinear conversion to the input video signal.

FIG. 3

EP 2 165 642 A1

**Description**

Technical Field

The present invention relates to an image processor for applying a color conversion process to color signals, a scope, and an endoscope apparatus including the same.

Background Art

In endoscope apparatuses, a scope having an image acquisition device is connected to a processor main unit, and the scope is inserted into a body and acquires an image of a subject to obtain a video signal. Various types of scopes are used for endoscope apparatuses depending on the intended image-acquisition use, and their characteristics are different depending on the type of scope, such as the spectral sensitivity characteristic of a CCD or the spectral transmisson characteristic of a color filter disposed in front of a CCD. Therefore, color reproduction of an acquired image is different depending on the type of scope. Color reproduction is a critical issue particularly in clinical settings where endoscope apparatuses are used.

Technologies for eliminating such differences in color reproduction and aimed at providing the same color reproduction among a plurality of different models of scopes include a color management system (hereinafter, referred to as "CMS"). In the CMS for conventional endoscope apparatuses, a color conversion process is performed by applying, in a processor, matrix conversion to a video signal output from a scope. At this time, conversion coefficients appropriate for the type of scope are written in a memory included in the processor. For example, Patent Citation 1 describes a technology in which an ID is held in a scope, color correction data appropriate for the ID is read from a memory when the scope is connected to a processor main unit, and a color correction process is performed based on the color correction data. Furthermore, Patent Citation 2 describes a technology for controlling a color correction matrix depending on a scope, to perform a color correction process.

Patent Citation 1:
Japanese Unexamined Patent Application, Publication No. Sho-62-199190

Patent Citation 2:
Japanese Unexamined Patent Application, Publication No. 2001-70240

Disclosure of Invention

In Patent Citations 1 and 2, since the color correction process is performed by using color correction data appropriate for a scope, an appropriate color correction process can be adaptively performed for each type of scope. However, even though the color correction matrix is optimized for each scope, since the degree of freedom of matrix conversion is low, color reproduction cannot be obtained at a sufficient accuracy level only through the optimization of matrix coefficients.

The present invention has been made in view of the above-described circumstances, and an object thereof is to provide an image processor that realizes a color conversion process appropriate for an image acquisition unit such as a scope and that can improve the accuracy of color reproduction; a scope for the image processor; and an endoscope apparatus including the same.

In order to solve the above-described problem, the present invention employs the following solutions.

According to a first aspect, the present invention provides an image processor including: a plurality of color conversion sections; an information acquisition section that acquires an index for selecting at least one of the plurality of color conversion sections as a specific color conversion means for applying a color conversion process to an input video signal; and a selection section that selects the at least one of the plurality of color conversion sections as the specific color conversion means, based on the index, in which the plurality of color conversion sections include a linear color conversion section that applies a color conversion process based on linear conversion to the input video signal and a nonlinear color conversion section that applies a color conversion process based on nonlinear conversion to the input video signal.

A feature of the linear color conversion process is that a high-speed and low-cost system can be built because its implementation is easy. On the other hand, a feature of the nonlinear color conversion process is that a high-quality video signal can be obtained because it can perform a higher-accuracy color conversion process than the linear color conversion process.

According to this aspect, it is possible to select at least one of the linear color conversion section and the nonlinear color conversion section as the specific color conversion means based on the index acquired by the information acquisition section, and to apply a color conversion process to an input video signal using the selected specific color conversion means.

In the above-described image processor, the index may be previously specified in each image acquisition unit that outputs the input video signal.

According to the above-described image processor, since the specific color conversion means is selected based on the index previously specified in each image acquisition unit, a color conversion process appropriate for the characteristics of the image acquisition unit can be performed.

In the above-described image processor, the index may be an ID number specified in an image acquisition unit that outputs the input video signal; and the selection section may have a table in which the ID number and a color conversion section to be selected are associated with each other and may select, by referring to the table, the color conversion section associated with the index as the specific color conversion means.

For example, the above-described ID number is a model number for identifying the model of an image acquisition unit. When a model number is used as the index in this way, existing information can be effectively utilized. Further, since a simple system is enabled by using the ID number as the index for selecting the specific color conversion means, it is possible to build a low-cost system that is easy to be implemented.

The above-described image processor may further include an external interface, in which the information acquisition section may acquire the index by reading the index recorded in a recording medium detachably connected to the external interface.

According to the above-described image processor, the specific color conversion means can be automatically selected by reading the index recorded in the recording medium.

In the above-described image processor, the index may be recorded in a recording medium accommodated in a scope detachably attached to a main body; and the information acquisition section may read the index from the recording medium while the scope is attached to the main body.

According to the above-described image processor, since the index can be read when the scope is attached thereto, a color conversion section appropriate for the scope is reliably selected.

In the above-described image processor, the recording medium may record information required for the color conversion process.

To perform the color conversion process, information to be referred to, such as matrix coefficients, needs to be recorded. When a table conversion process, for example, is performed as the nonlinear color conversion process, a large storage capacity is required to record table coefficients to be referred to. Therefore, a recording medium having a large storage capacity is required in order to record, in the image processor, table coefficients for each image acquisition unit.

According to the above-described image processor, on the other hand, table coefficients for each image acquisition unit are recorded in a recording medium to be connected to the external interface or in a recording medium included in the scope, thereby allowing a reduction in the storage capacity of the image processor.

The above-described image processor may further include an input section that is used by a user to input the index, in which the information acquisition section may acquire the index input through the input section.

According to the above-described image processor, the user can select a desired specific color conversion means.

In the above-described image processor, the nonlinear color conversion section may apply the color conversion process based on table conversion.

When the color conversion process is performed based on table conversion, a higher-quality video signal can be obtained.

According to a second aspect, the present invention provides a scope that is detachably attached to one of the above-described image processors and that accommodates a recording medium having the index recorded therein.

According to this aspect, the image processor can select a color conversion process appropriate for the characteristics of the scope. Further, information such as linear matrix coefficients used for the color conversion process is output to the image processor, thereby allowing a reduction in the storage capacity of the image processor.

According to a third aspect, the present invention provides an endoscope apparatus including: one of the above-described image processors; and the above-described scope.

According to this aspect, a color conversion process appropriate for the characteristics of each scope can be performed by selecting specific color conversion means based on the index previously specified for the scope, thereby allowing stable image color reproduction.

According to the present invention, since it is possible to select at least one of the linear color conversion section and the nonlinear color conversion section as the specific color conversion means based on the index acquired by the information acquisition section and to apply a color conversion process to an input video signal by the selected specific color conversion means, an advantage is afforded in that the color conversion process appropriate for an image acquisition unit such as a scope can be realized, and the accuracy of color reproduction can be improved.

Brief Description of Drawings

[FIG. 1] FIG. 1 is a functional block diagram showing, in expanded fashion, the functions of an endoscope apparatus according to a first embodiment of the present invention.

[FIG. 2] FIG. 2 is an explanatory diagram of a color-difference line-sequential complementary color filter of the endoscope apparatus shown in FIG. 1.

[FIG. 3] FIG. 3 is a diagram of a first configuration of a color conversion section of the endoscope apparatus shown in FIG. 1.

[FIG. 4] FIG. 4 is a diagram of a second configuration of the color conversion section of the endoscope apparatus shown in FIG. 1.

[FIG. 5] FIG. 5 is a configuration diagram of a determination section shown in FIG. 3.

[FIG. 6] FIG. 6 is a diagram of a third configuration of the color conversion section of the endoscope apparatus shown in FIG. 1.

[FIG. 7] FIG. 7 is a flowchart of a color conversion process performed in the endoscope apparatus shown in FIG. 1.

[FIG. 8] FIG. 8 is a functional block diagram showing, in expanded fashion, the functions of an endoscope apparatus according to a second embodiment of the present invention.

[FIG. 9] FIG. 9 is a diagram of a first configuration of a color conversion section of the endoscope apparatus shown in FIG. 8.

[FIG. 10] FIG. 10 is an explanatory diagram of a processing method table ROM shown in FIG. 9.

[FIG. 11] FIG. 11 is a diagram of a second configuration of the color conversion section of the endoscope apparatus shown in FIG. 8.

Explanation of Reference:

1, 10: endoscope apparatus
101: scope
104: memory
105: connecting section
115: color conversion section
120: input section
121: processor section
201: determination section
202: linear matrix conversion section
203: table conversion section
209: nonlinear conversion section
250: information acquisition section
251: selection section

Best Mode for Carrying Out the Invention

First Embodiment

An image processor, a scope, and an endoscope apparatus including the same according to a first embodiment of the present invention will be described below with reference to the drawings.

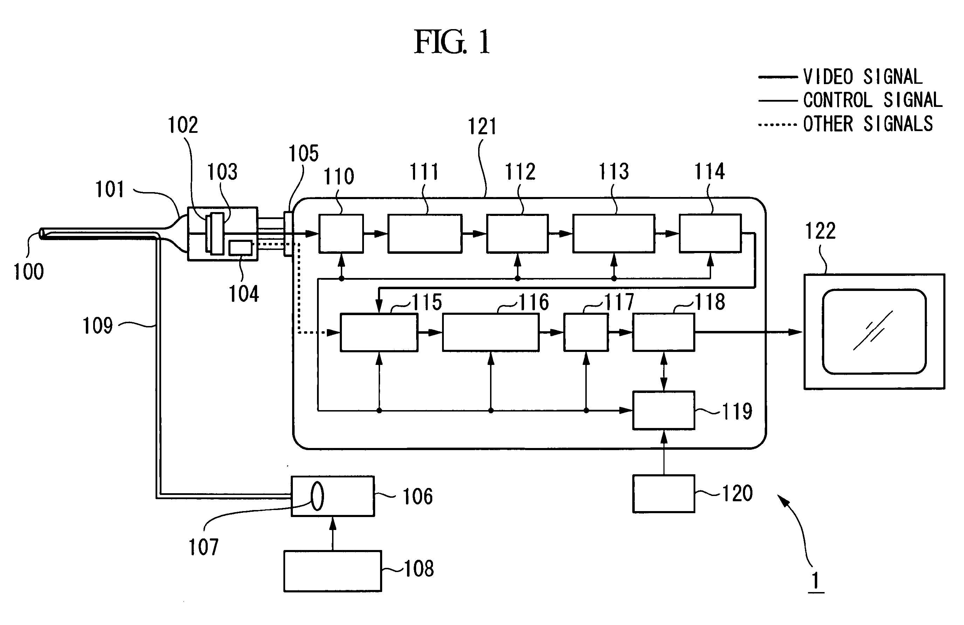

FIG. 1 is a functional block diagram showing, in expanded fashion, the functions of the endoscope apparatus according to this embodiment.

An endoscope apparatus 1 includes, as main components, a processor section (image processor) 121 that applies image processing to input images and a scope (image acquisition unit) 101 that is detachably attached to the processor section 121 and that includes an image acquisition device.

The scope 101 accommodates a lens system 100, a color filter 102, a CCD 103, and a memory (recording medium) 104 that stores an ID number for identifying a color conversion processing method for the model of each scope 101 and other data.

Furthermore, the scope 101 is connected to a light source section 106 by a light guide 109. The light source section 106 has a lamp 107 and emits light having a light level specified by a light-level control section 108.

The processor section 121 includes an A/D 110, a buffer 111, an interpolation section 112, a Y/C separation section 113, a WB section 114, a color conversion section 115, a signal processing section 116, a D/A 117, an output section 118, and a control section 119.

The A/D 110, which is connected to the scope 101 via a connecting section 105, is connected to the buffer 111. The buffer 111 is connected to the interpolation section 112, and the interpolation section 112 is connected to the Y/C separation section 113. In addition, the Y/C separation section 113 is connected to the WB section 114. Furthermore, the WB section 114 is connected to the color conversion section 115, and the color conversion section 115 is connected to the signal processing section 116. The signal processing section 116 is connected to the D/A 117, and the D/A 117 is connected to the output section 118. The output section 118 is connected to an external display unit 122.

Furthermore, the control section 119, which is a microcomputer, for example, is bi-directionally connected to the A/D 110, the interpolation section 112, the Y/C separation section 113, the WB section 114, the color conversion section 115, the signal processing section 116, the D/A 117, and the output section 118. A power switch and an input section 120 that is used by a user to switch various settings for photographing are also bi-directionally connected to the control section 119.

The operation of the thus-configured endoscope apparatus 1 will be described below.

First, a color mode is specified by the user via the input section 120, and the level of light from the light source is specified in the light-level control section 108. Then, the lamp 107 included in the light source section 106 emits light, and the light is supplied to the scope 101 via the light guide 109 to irradiate a subject. A video of the subject irradiated with the light in this way is acquired by the scope 101 and is sent to the processor section 121 as a video signal.

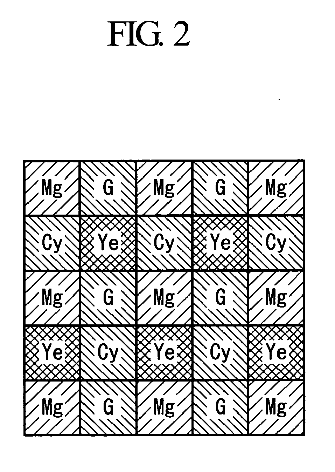

Note that, in this embodiment, is it assumed that the image acquisition system is a single-plane CCD in front of which a color-difference line-sequential complementary color filter is disposed. In a color-difference line-sequential system, 2 x 2 pixels are handled as a base unit, and Cy (cyan), Mg (magenta), Ye (yellow), and G (green) are arrayed at the respective pixels, as shown in FIG. 2. However, the positions of Cy and Ye are reversed in each line in this embodiment. Furthermore, in this embodiment, the bit length of the digitized video signal is 12 bits, for example.

The flow of the video signal in the processor section 121 will be described below.

The video signal acquired by the scope 101 is sent to the A/D 110, the buffer 111, the interpolation section 112, the Y/C separation section 113, the WB section 114, and the color conversion section 115, in that order, and is subjected to a color conversion process in the color conversion section 115. The video signal that has been subjected to the color conversion process in the color conversion section 115 is sent to the signal processing section 116, the D/A 117, and the output section 118, in that order, and is output by the output section 118 to the display unit 122.

Video signal processing performed in the processor section 121 will be described below in detail.

The video signal that has been converted into a digital signal in the A/D 110 is sent to the interpolation section 112 via the buffer 111. In the interpolation section 112, a four-plane video signal to which a known interpolation process has been applied is generated and transferred to the Y/C separation section 113. The Y/C separation section 113 calculates luminance and color-difference signals from the video signal obtained via the interpolation section 112. The luminance and color-difference signals are calculated for each pixel, as in Equation (1).

[EXPRESSION 1]

$$
\begin{bmatrix} Y_i \\ Cb_i \\ Cr_i \end{bmatrix} = \begin{bmatrix} m_1 & m_2 & m_3 & m_4 \\ m_5 & m_6 & m_7 & m_8 \\ m_9 & m_{10} & m_{11} & m_{12} \end{bmatrix} \begin{bmatrix} Cy_i \\ Mg_i \\ Ye_i \\ G_i \end{bmatrix} \quad \cdots \text{ (1)}
$$

wherein i indicates the coordinate of a pixel, and $m_1$ to $m_{12}$ indicate matrix coefficients used to convert Cy, Mg, Ye, and G signals into Y, Cb, and Cr signals. This calculation is performed for all pixels. The YCbCr signals calculated by Equation (1) are transferred to the WB section 114.

The WB section 114 performs a white balance process by multiplying the color-difference signals Cb and Cr by predetermined white-balance coefficients. The YCbCr signals obtained after the white balance process are transferred to the color conversion section 115. The color conversion section 115 determines a color conversion processing method appropriate for the scope 101 based on an ID number that is recorded in the memory 104 included in the scope 101 and that identifies the color conversion processing method for each scope 101, and applies a color conversion process to the YCbCr signals.

The YCbCr signals that have been subjected to the color conversion process are transferred to the signal processing section 116. The signal processing section 116 converts the YCbCr signals into RGB signals through a known color space conversion process, further applies a known gradation conversion process, a known edge enhancement process, etc. to the RGB signals, and transfers them to the D/A 117. The D/A 117 converts the RGB signals obtained via the signal processing section 116 into analog signals and transfers the converted RGB signals to the output section 118. The output section 118 displays the RGB signals obtained via the D/A 117, on the display unit 122.

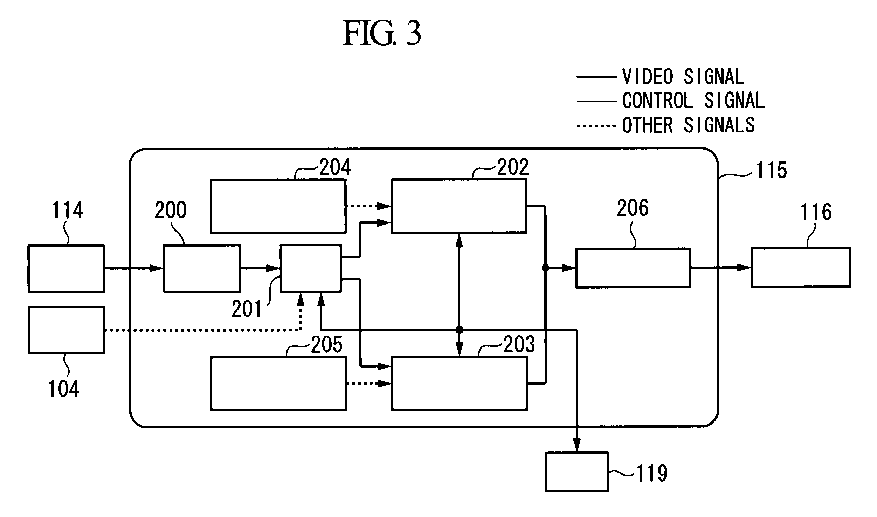

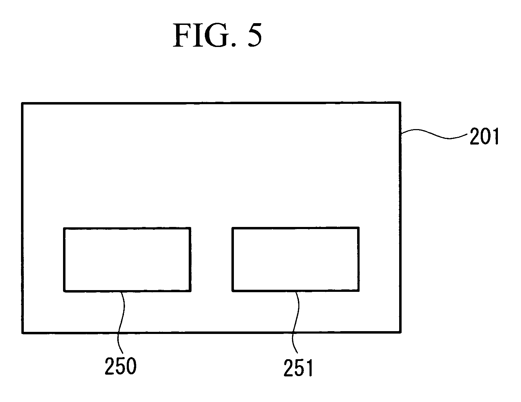

The color conversion process performed in the color conversion section 115 will be described below in detail. FIG. 3 is a functional block diagram showing a first configuration example of the color conversion section 115. As shown in FIG. 3, the color conversion section 115 includes a buffer 200, a determination section 201, a linear matrix conversion section (linear color conversion section) 202, a table conversion section (nonlinear color conversion section) 203, a linear matrix coefficient ROM 204, a table coefficient ROM 205, and an image buffer 206. As shown in FIG. 5, the determination section 201 has an information acquisition section 250 and a selection section 251.

The buffer 200 connected to the WB section 114 is connected to the determination section 201, which is connected to the memory 104. The determination section 201 is connected to the linear matrix conversion section 202 and the table conversion section 203. The linear matrix coefficient ROM 204 is connected to the linear matrix conversion section 202, and the table coefficient ROM 205 is connected to the table conversion section 203. Also, the linear matrix conversion section 202 and the table conversion section 203 are connected to the image buffer 206. Furthermore, the image buffer 206 is connected to the signal processing section 116.

Note that the control section 119 is bi-directionally connected to the determination section 201, the linear matrix conversion section 202, and the table conversion section 203.

YCbCr signals transferred from the WB section 114 are temporarily stored in the buffer 200 and are transferred to the determination section 201. Further, the ID number recorded in the memory 104 included in the scope 101 is transferred to the determination section 201. In the determination section 201, a color conversion processing method is determined based on the ID number obtained via the memory 104. The color conversion processing method is determined by using Equation (1-1), for example.

[EXPRESSION 2]

$$\begin{cases} if \quad ID = 1 \quad method1 \\ if \quad ID = 2 \quad method2 \end{cases} \quad \cdots \quad (1-1)$$

wherein method1 and method2 indicate predetermined different color conversion processing methods. In this embodiment, it is assumed that method1 indicates a color conversion processing method based on linear matrix conversion and method2 indicates a color conversion processing method based on table conversion, for example. The determination section 201 transfers the YCbCr signals transferred from the buffer 200 to the linear matrix conversion section 202 when the ID number is 1, and transfers the YCbCr signals to the table conversion section 203 when the ID number is 2.

First, a description will be given below of a case where the ID number is 1, that is, a case where a linear color conversion process is performed.

The linear matrix conversion section 202 reads, for each pixel, YCbCr signals transferred from the determination section 201 and performs linear matrix conversion shown in Equation (2).

[EXPRESSION 3]

$$\begin{bmatrix} Y_i' \\ Cb_i' \\ Cr_i' \end{bmatrix} = \begin{bmatrix} a_1 & a_2 & a_3 \\ a_4 & a_5 & a_6 \\ a_7 & a_8 & a_9 \end{bmatrix} \begin{bmatrix} Y_i \\ Cb_i \\ Cr_i \end{bmatrix} \quad \cdots \quad (2)$$

wherein $Y_i$, $Cb_i$, and $Cr_i$ indicate input YCbCr signals of pixel i, and $Y_i'$, $Cb_i'$, $Cr_i'$ indicate YCbCr signals of pixel i obtained after the linear matrix conversion. Further, $a_1$ to $a_9$ indicate linear matrix coefficients.

The linear matrix coefficient ROM 204 records linear matrix coefficients in advance, and the linear matrix conversion section 202 reads predetermined linear matrix coefficients from the linear matrix coefficient ROM 204 and performs the linear matrix conversion. The linear matrix conversion is performed with the aim of providing color reproduction that is the same as that of the target scope, and functions to reduce numerical errors with respect to the target YCbCr signals. The difference in color reproduction between scopes is caused because the spectral characteristics of image acquisition devices included in the scopes are different. Therefore, it is possible to come close to the target color reproduction by calculating linear matrix coefficients that eliminate the difference between the spectral characteristics of the two image acquisition devices included in the scope that provides the target color reproduction and in the scope that is subjected to the color conversion process, and by applying the linear matrix conversion to the luminance and color-difference signals output from the scope to be processed. Thus, for example, the linear matrix coefficients are calculated by using the least-squares method such that the numerical square error becomes minimum at each wavelength between the target spectral characteristic and the spectral characteristic obtained after the linear matrix conversion. Here, the matrix coefficients $a_1$ to $a_9$ that minimize E of Equation (3) are calculated by the least-squares method. Note that the range of $\Sigma$ for $\lambda$ is specified from 380 to 780 nm; the range of $\lambda$ can be changed as desired.
[EXPRESSION 4]

$$\sum_\lambda \left( \begin{bmatrix} S1_Y(\lambda) \\ S1_{Cb}(\lambda) \\ S1_{Cr}(\lambda) \end{bmatrix} - \begin{bmatrix} a_1 & a_2 & a_3 \\ a_4 & a_5 & a_6 \\ a_7 & a_8 & a_9 \end{bmatrix} \begin{bmatrix} S2_Y(\lambda) \\ S2_{Cb}(\lambda) \\ S2_{Cr}(\lambda) \end{bmatrix} \right)^2 \quad \cdots \quad (3)$$

wherein $S1_Y(\lambda)$, $S1_{Cb}(\lambda)$, and $S1_{Cr}(\lambda)$ indicate the spectral characteristics of the Y signal, the Cb signal, and the Cr signal of the image acquisition device that provides the target color reproduction. Further, $S2_Y(\lambda)$, $S2_{Cb}(\lambda)$, and $S2_{Cr}(\lambda)$ indicate the spectral characteristics of the Y signal, the Cb signal, and the Cr signal of the image acquisition device that is subjected to the color conversion process.
The YCbCr' signals to which the linear matrix conversion has been applied as described above are transferred to the image buffer 206. After the linear matrix conversion is applied to the YCbCr signals of all pixels, the YCbCr' signals stored in the image buffer 206 are transferred to the signal processing section 116.
Next, a description will be given below of a case where the ID number is 2, that is, a case where a table conversion process is performed.
The table conversion section 203 reads, for each pixel, the YCbCr signals transferred from the determination section 201 and applies a color conversion process thereto with reference to table coefficients recorded in the table coefficient ROM 205 based on a combination of the YCbCr signals of each pixel.
The table coefficient ROM 205 records, in advance, an associated relationship between input YCbCr signals and output YCbCr' signals. The table coefficients can be obtained, for example, by applying nonlinear matrix conversion that handles high-order terms of Y, Cb, and Cr signals, in addition to the linear matrix conversion shown in Equation (2), to each combination of Y, Cb, and Cr signals and by generating a table in which the input Y, Cb, and Cr signals and the converted Y, Cb, and Cr signals are associated in a one-to-one manner.

Further, it is also possible to generate the table by associating with each other the video signals for each of predetermined color charts, acquired and output from an image acquisition unit providing the target color reproduction and an image acquisition unit subjected to the color conversion process.

The YCbCr' signals converted by the table conversion section 203 are transferred to the image buffer 206 and are stored therein. After the color conversion process is applied to YCbCr signals of all pixels, the YCbCr' signals stored in the image buffer 206 are transferred to the signal processing section 116.

As described above, according to the endoscope apparatus of this embodiment, it is possible to select at least one of the linear matrix conversion section 202 and the table conversion section 203 based on an index obtained by the determination section 201 and to apply the color conversion process to an input video signal using the selected color conversion processing method. Further, by selecting specific color conversion means based on the index previously set in each scope, a color conversion process appropriate for the characteristics of the scope can be performed.

Note that, in the above-described embodiment, a configuration is used in which the memory 104 is accommodated in the scope 101 and the determination section 201 reads an index with the scope 101 attached to the processor section 121; however, the configuration for reading an index is not limited to this example. For example, a configuration may be used in which an external interface (not shown) to which a recording medium that records an index for the scope in advance is detachably connected is provided on the processor section 121 and, when the recording medium is connected to the external interface, the determination section reads the index from the recording medium. The recording medium needs to be a computer-readable medium and can be, for example, a USB memory, an SD memory, a flash memory, a CD-ROM, or the like.

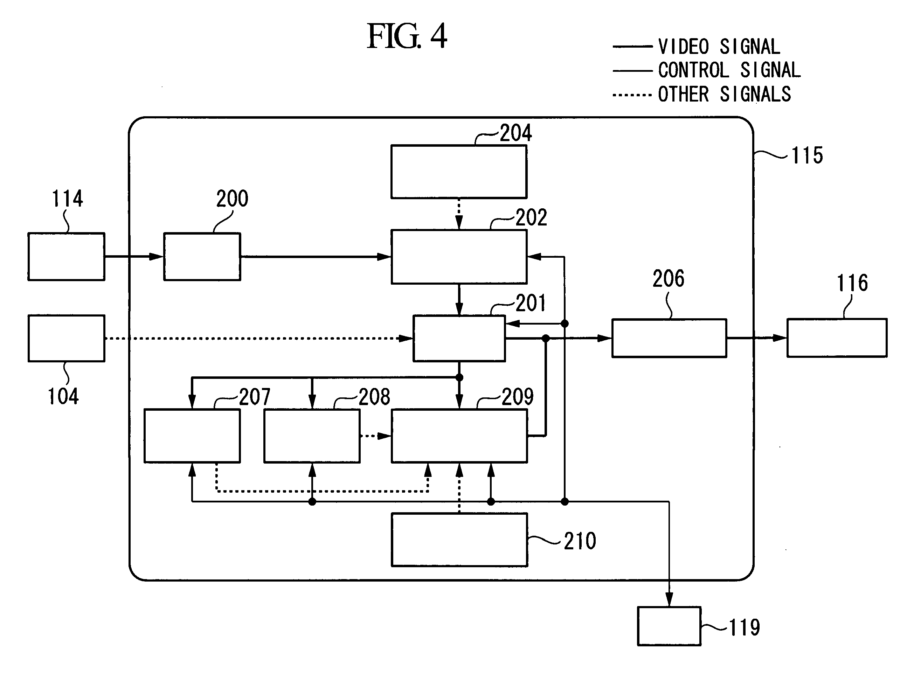

FIG. 4 shows a second configuration example of the color conversion section 115, in which the table conversion section 203 and the table coefficient ROM 205 shown in FIG. 3 are omitted, and a hue calculation section 207, a chroma calculation section 208, a nonlinear conversion section 209, and a nonlinear conversion coefficient ROM 210 are added. The basic configuration is the same as that of the color conversion section 115 shown in FIG. 3, and identical names and reference numerals are assigned to identical structures. The differences will be mainly described below.

In the color conversion section 115 shown in FIG. 4, the buffer 200 is connected to the linear matrix conversion section 202, and the linear matrix conversion section 202 is connected to the determination section 201. Furthermore, the linear matrix coefficient ROM 204 is connected to the linear matrix conversion section 202.

Furthermore, the determination section 201 is connected to the image buffer 206, the hue calculation section 207, the chroma calculation section 208, and the nonlinear conversion section 209. The hue calculation section 207 and the chroma calculation section 208 are connected to the nonlinear conversion section 209, and the nonlinear conversion section 209 is connected to the image buffer 206. Furthermore, the nonlinear conversion coefficient ROM 210 is connected to the nonlinear conversion section 209.

The control section 119 is bi-directionally connected to the determination section 201, the linear matrix conversion section 202, the hue calculation section 207, the chroma calculation section 208, and the nonlinear conversion section 209.

The YCbCr signals transferred from the WB section 114 are temporarily stored in the buffer 200 and are transferred to the linear matrix conversion section 202. The linear matrix conversion section 202 applies linear matrix conversion to the YCbCr signals of each pixel i that are obtained via the buffer 200, based on Equation (2).

In this way, the linear matrix conversion is applied to the YCbCr signals of all pixels, and the converted YCbCr' signals are transferred to the determination section 201. The determination section 201 determines a color conversion processing method based on an ID number obtained via the memory 104. For example, the determination is performed by using Equation (4).

[EXPRESSION 5]

$$\begin{cases} if & ID = 1 & method1 \\ if & ID = 3 & method3 \end{cases} \quad \cdots (4)$$

wherein method3 indicates a color conversion processing method of further applying nonlinear conversion to the YCbCr' signals to which the linear matrix conversion has been applied. When the ID number is 1, the YCbCr' signals are transferred to the image buffer 206, and, when the ID number is 3, the YCbCr' signals are transferred to the hue calculation section 207, the chroma calculation section 208, and the nonlinear conversion section 209. The hue calculation section 207 calculates a hue signal based on Equation (5).

[EXPRESSION 6]

$$H_i = \tan^{-1}(Cb'_i / Cr'_i) \qquad \cdots (5)$$

wherein $H_i$ indicates a hue signal of the pixel i, and $Cr_i'$ and $Cb_i'$ indicate Cr and Cb signals of the pixel i that are obtained after the linear matrix conversion. Furthermore, $H_i$ falls within the range of values 0 to 359. The calculated hue signal is transferred to the nonlinear conversion section 209. The chroma calculation section 208 calculates a chroma signal based on Equation (6).

[EXPRESSION 7]

$$C_i = \sqrt{Cr_i'^2 + Cb_i'^2} \qquad \cdots (6)$$

wherein $C_i$ indicates a chroma signal of the pixel i. The calculated chroma signal is transferred to the nonlinear conversion section 209. The nonlinear conversion section 209 applies a color conversion process based on a nonlinear operation, only to YCbCr' signals belonging to a predetermined particular color region that is defined in advance based on the hue signal and the chroma signal obtained via the hue calculation section 207 and the chroma calculation section 208. The nonlinear operation is performed using the following equations, for example.

[EXPRESSION 8]

$$Y_i'' = q \cdot Y_i'^2 + r \cdot Cb_i'^2 + s \cdot Cr_i'^2$$

$$Cb_i'' = t \cdot Y_i'^2 + u \cdot Cb_i'^2 + v \cdot Cr_i'^2 \qquad \cdots (7)$$

$$Cr_i'' = w \cdot Y_i'^2 + x \cdot Cb_i'^2 + y \cdot Cr_i'^2$$

wherein q to y indicate predetermined coefficients for the nonlinear operation. The nonlinear conversion coefficient ROM 210 previously records the coefficients q to y, and the nonlinear conversion section 209 reads the predetermined coefficients from the nonlinear conversion coefficient ROM 210 and performs the color conversion process based on the nonlinear operation.

Note that the color conversion process is performed based on the high-order nonlinear operation in the example described above; however, it is also possible to perform nonlinear matrix conversion that handles high-order terms of the Y, Cb, and Cr signals in addition to the linear matrix conversion shown in Equation (2), for example.

According to the endoscope apparatus of this embodiment, it is possible to select at least one of the linear matrix conversion section 202 and the nonlinear conversion section 209 based on an index obtained by the determination section 201 and to apply a color conversion process to an input video signal using the selected color conversion processing method. Furthermore, by selecting specific color conversion means based on the index previously set in each scope, a color conversion process appropriate for the characteristics of the scope can be performed.

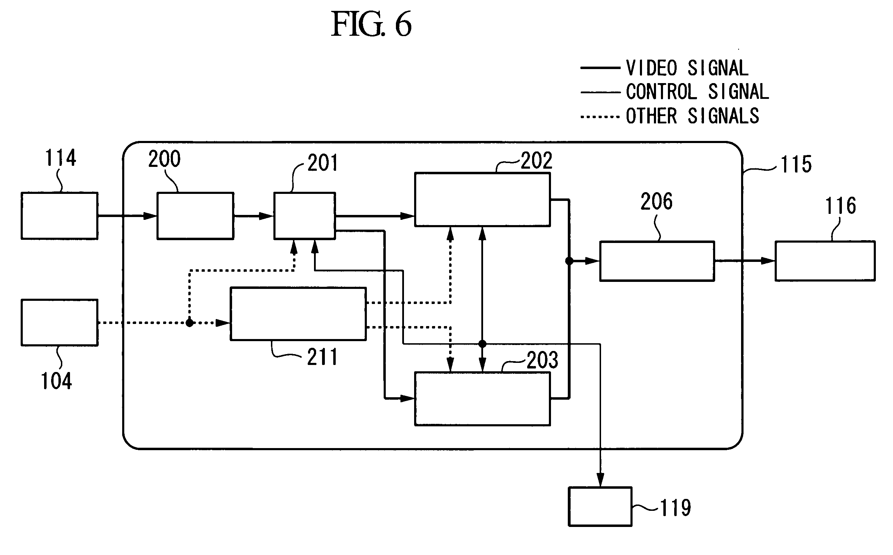

Note that, in the above-described configuration example, coefficients used for the color conversion processes are held in the ROMs in the processor; however, the present invention is not limited to this example, and a configuration such as that shown in FIG. 6 can also be used, for example. FIG. 6 shows a configuration in which the linear matrix coefficient ROM 204 and the table coefficient ROM 206 are omitted from the configuration diagram of the color conversion section 115 of FIG. 3. The basic configuration is the same as that of the color conversion section 115 shown in FIG. 3, and identical names and reference numerals are assigned to identical structures. The differences will be mainly described below.

The memory 104 is connected to a coefficient buffer 211. The coefficient buffer 211 is connected to the linear matrix conversion section 202 and the table conversion section 203.

The memory 104 previously records an ID number serving as the index for determining a color conversion processing method, and predetermined coefficients used for that color conversion processing method, for example, either linear matrix coefficients or table coefficients. The ID number recorded in the memory 104 is transferred to the determination section 201. The determination section 201 determines a color conversion processing method using Equation (1-1) based on the ID number obtained via the memory 104.

The determination section 201 transfers the YCbCr signals transferred from the buffer 200 to the linear matrix conversion section 202 when the ID number is 1, and transfers the YCbCr signals to the table conversion section 203 when the ID number is 2. The linear matrix conversion section 202 reads, for each pixel, the YCbCr signals transferred from the determination section 201 and performs linear matrix conversion shown in Equation (2). At this time, the linear matrix conversion section 202 reads predetermined linear matrix coefficients from the coefficient buffer 211 and performs linear matrix conversion. The table conversion section 203 reads, for each pixel, the YCbCr signals transferred from the determination section 201 and performs the color conversion process with reference to table coefficients recorded in the coefficient buffer 211 based on a combination of the YCbCr signals of each pixel. Whether to perform either the linear matrix conversion or the table conversion is determined depending on the model of each scope. The subsequent processes are the same as those described with reference to FIG. 3.

As described above, according to the endoscope apparatus of this configuration example, since it is unnecessary to provide the linear matrix coefficient ROM 204, the table coefficient ROM 206, and the nonlinear conversion coefficient ROM 210 in the color conversion section 115 by recording table coefficients for each scope in its memory 104, the storage capacity of the image processor can be reduced.

Second Embodiment

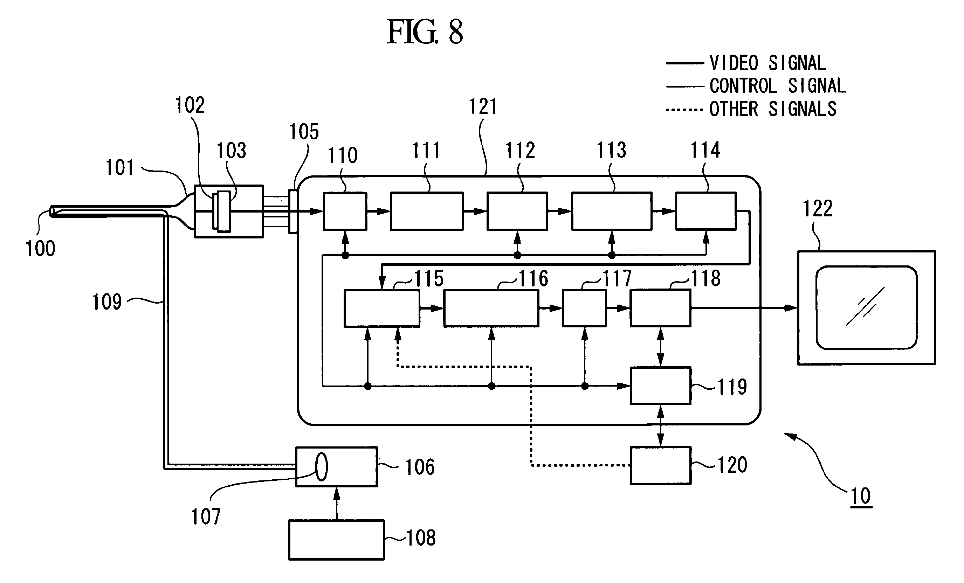

Next, a second embodiment of the present invention will be described by mainly using FIG. 8.

An image processor, an image acquisition unit, and an endoscope apparatus including the same according to this embodiment are different from those of the first embodiment in that an index for selecting at least one of the linear color conversion means and the nonlinear color conversion means is manually set by a user. For the endoscope apparatus according to this embodiment, the differences from the first embodiment will be mainly described, and a description of similarities will be omitted.

FIG. 8 is a functional block diagram showing, in expanded fashion, the functions of the endoscope apparatus according to this embodiment.

In this embodiment, a configuration in which the memory 104 is omitted from the configuration of the first embodiment is used. The basic configuration is the same as that of the first embodiment, and identical names and reference numerals are assigned to identical structures. The input section 120 is connected to the color conversion section 115.

The flow of a video signal in an endoscope apparatus 10 having the above-described configuration will be described below.

When the user operates the input section 120 and specifies a color mode and a model number for identifying the scope model, the specified color mode is transferred to the control section 119, and the specified model number is transferred to the color conversion section 115.

On the other hand, in the scope, the level of light from the light source is specified by the light-level control section 108, and a video of a subject is acquired by the scope. The video signal acquired by the scope is input to the processor main unit, is subjected to predetermined image processing, and is then input to the color conversion section 115. The determination section of the color conversion section 115 determines a color conversion processing method based on the model number input through the input section and performs a color conversion process using the determined color conversion processing method. The subsequent processes are performed in the same way as in the first embodiment.

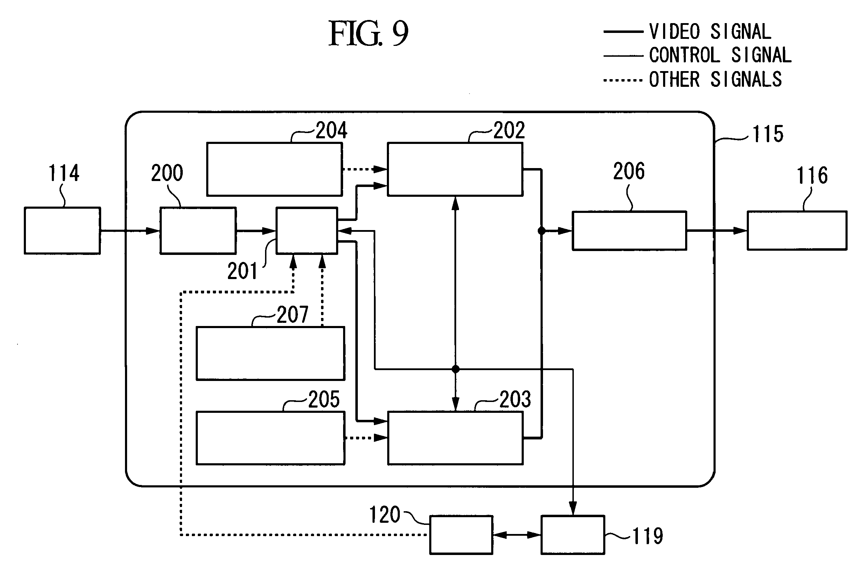

FIG. 9 shows a configuration example of the color conversion section 115, in which the memory 104 shown in FIG. 3 is omitted and a processing method table ROM 207 is added. The basic configuration is the same as that of the color conversion section 115 shown in FIG. 3, and identical names and reference numerals are assigned to identical structures.

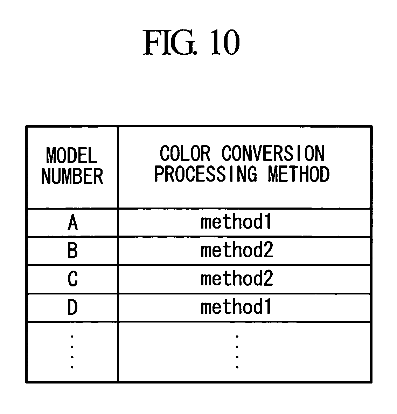

The processing method table ROM 207 is connected to the determination section 201. The control section 119 is bi-directionally connected to the input section 120. YCbCr signals transferred from the WB section 114 are temporarily stored in the buffer 200 and are transferred to the determination section 201. The determination section 201 determines a color conversion processing method with reference to a table coefficient recorded in the processing method table ROM 207 based on the model number of the scope obtained via the input section 120. As shown in FIG. 10, for example, the processing method table ROM 207 records table coefficients that describe the combinations of the model numbers of scopes and the color conversion processing methods corresponding thereto. The subsequent processes are performed in the same way as in the color conversion section 115 shown in FIG. 3.

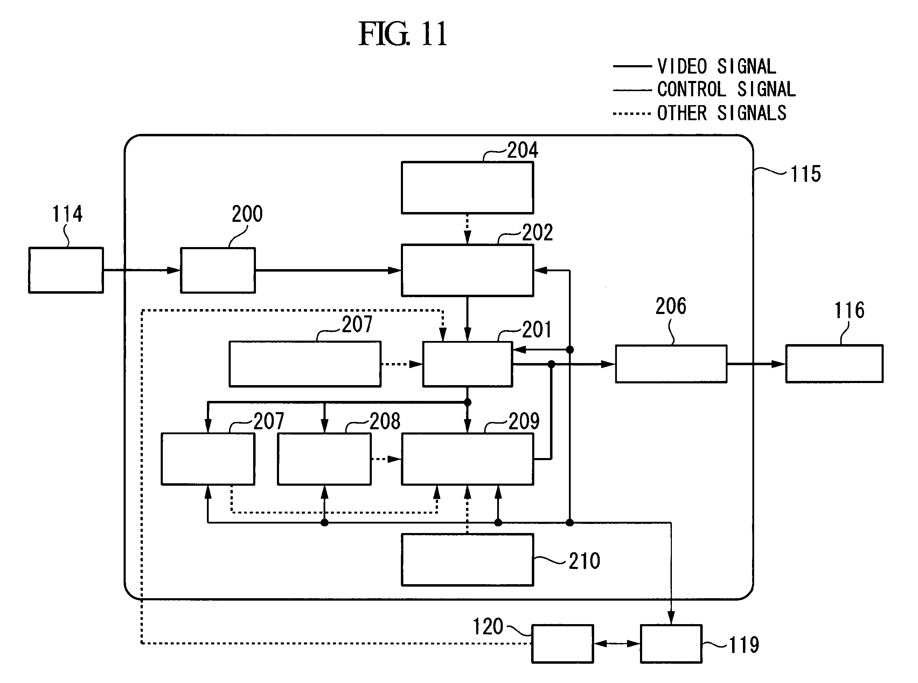

FIG. 11 shows a configuration example of the color conversion section 115, in which the memory 104 shown in FIG. 4 is omitted and the processing method table ROM 207 is added. The basic configuration is the same as that of the color conversion section 115 shown in FIG. 4, and identical names and reference numerals are assigned to identical structures.

The input section 120 is connected to the determination section 201. The processing method table ROM 207 is connected to the determination section 201. The control section 119 is bi-directionally connected to the input section 120. YCbCr signals that have been subjected to the linear matrix conversion in the linear matrix conversion section 202 are transferred to the determination section 201. The determination section 201 determines a color conversion processing method with reference to a table coefficient recorded in the processing method table ROM 207 based on the model number of the scope obtained via the input section 120. The subsequent processes are performed in the same way as in the color conversion section 115 shown in FIG. 4.

According to the image processor of this embodiment, since the user can specify a model number, even when a scope having no memory is used, a color conversion processing method suitable for the scope can be selected.

Note that, in the above-described configuration example, a color conversion processing method appropriate for the model number of a scope is determined by externally inputting the model number of the scope; however, the present invention is not limited to this example, and, for example, a configuration can also be used in which a menu listing model numbers is displayed on the display unit when a scope is connected to the processor section, and the user manually selects the model number of the scope from the menu.

The processing is performed by hardware in the respective embodiments described above; however, the present invention does not need to be limited to such a configuration, and a configuration in which the processing is performed by separate software can also be used.

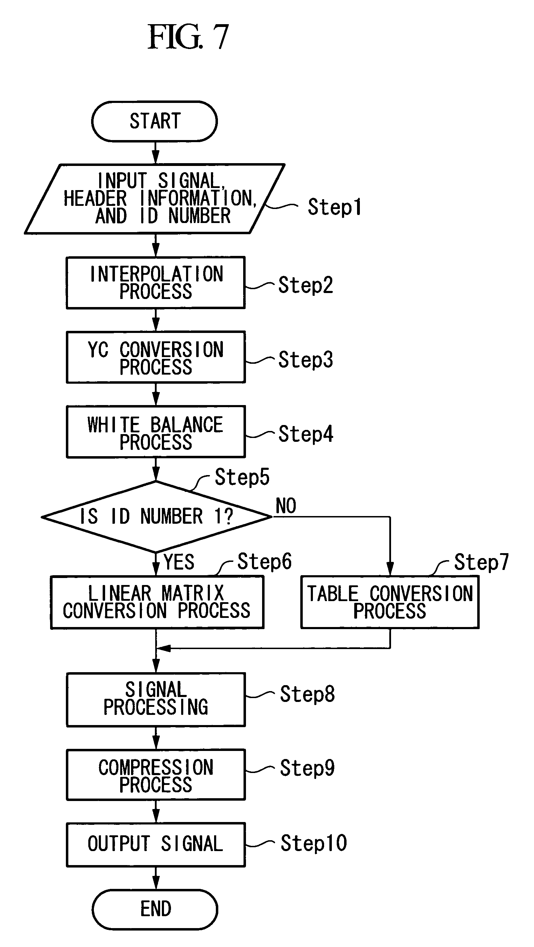

FIG. 7 shows a flow of software processing in the endoscope apparatus in the first embodiment.

In Step 1, an unprocessed video signal, an ID number for determining a color conversion processing method, and header information that includes accompanying information on image acquisition conditions, such as a white balance coefficient, are read. In Step 2, an interpolation process is performed to generate a four-plane video signal. In Step 3, the interpolated video signal obtained in Step 2 is converted into YCbCr signals. In Step 4, a white balance process is applied to the YCbCr signals. In Step 5, it is determined whether the ID number input in Step 1 is 1. If the ID number is 1, the flow advances to Step 6. If the ID number is not 1, the flow advances to Step 7. In Step 6, a color conversion process based on linear matrix conversion is applied to the YCbCr signals obtained in Step 5. In Step 7, a color conversion process based on table conversion is applied to the YCbCr signals obtained in Step 5. In Step 8, a known gradation conversion process, a known edge enhancement process, etc. are performed. In Step 9, a known compression process, e.g., JPEG is performed. In Step 10, the processed signals are output to a recording apparatus such as a hard disk and are recorded therein, and the process thus ends.

In the embodiments described above, the image acquisition system is a complementary single-plane CCD; the present invention is not limited to this, and, for example, a primary-color single-plane CCD or three-plane CCD can also be used. Further, the present invention can be applied not only to a CCD but also to a CMOS device.

Further, in the embodiments described above, a configuration is used in which one color conversion processing method is determined from two color conversion processing methods, as shown in FIGS. 3 and 4, for example; however, the present invention is not limited to this configuration and, for example, a configuration can also be used in which one color conversion processing method is selected from among three color conversion processing methods. In that case, a second determination section may be provided at the subsequent stage of the linear matrix conversion section 202 shown in FIG. 3. First, the determination section 201 determines whether to use a color conversion processing method based on table conversion. When it is determined to use a color conversion processing method based on linear matrix conversion, the second determination section provided at the subsequent stage of the linear matrix conversion section 202 determines whether to use a color conversion processing method based on nonlinear conversion.

Further, in the above-described embodiments, a description has been given of an example case where the scope is used as an image acquisition unit; however, the present invention is not limited to this example case. For example, in a case where a color conversion process is applied to a video signal acquired by a different model of digital camera or other cases, the above-described processor main unit may be used.

**Claims**

1.  An image processor comprising:

    a plurality of color conversion sections;
    an information acquisition section that acquires an index for selecting at least one of the plurality of color conversion sections as a specific color conversion means for applying a color conversion process to an input video signal; and
    a selection section that selects the at least one of the plurality of color conversion sections as the specific color conversion means, based on the index,
    wherein the plurality of color conversion sections comprise a linear color conversion section that applies a color conversion process based on linear conversion to the input video signal and a nonlinear color conversion section that applies a color conversion process based on nonlinear conversion to the input video signal.

2.  An image processor according to claim 1, wherein the index is previously specified in each image acquisition unit that outputs the input video signal.

3.  An image processor according to claim 1 or 2, wherein:

    the index is an ID number specified in an image acquisition unit that outputs the input video signal; and
    the selection section has a table in which the ID number and a color conversion section to be selected are associated with each other and selects, by referring to the table, the color conversion section associated with the index as the specific color conversion means.

4.  An image processor according to one of claims 1 to 3, further comprising an external interface,
    wherein the information acquisition section acquires the index by reading the index recorded in a recording medium detachably connected to the external interface.

5.  An image processor according to one of claims 1 to 3, wherein:

    the index is recorded in a recording medium accommodated in a scope detachably attached to a main body; and
    the information acquisition section reads the index from the recording medium while the scope is attached to the main body.

6.  An image processor according to claim 4 or 5, wherein the recording medium records information required for the color conversion process.

7.  An image processor according to one of claims 1 to 6, further comprising an input section that is used by a user to input the index,
    wherein the information acquisition section acquires the index input through the input section.

8.  An image processor according to one of claims 1 to 7, wherein the nonlinear color conversion section applies the color conversion process based on table conversion.

9.  A scope that is detachably attached to an image processor according to one of claims 1 to 8 and that accommodates a recording medium having the index recorded therein.

10. An endoscope apparatus comprising:

    an image processor according to one of claims 1 to 8; and
    a scope according to claim 9.

# FIG. 1

EP 2 165 642 A1

# FIG. 2

# FIG. 3

─── VIDEO SIGNAL
── CONTROL SIGNAL
······ OTHER SIGNALS

EP 2 165 642 A1

# FIG. 4

EP 2 165 642 A1

VIDEO SIGNAL
CONTROL SIGNAL
OTHER SIGNALS

204

115

114

200

202

206

116

104

201

207  208  209

210

119

# FIG. 5

## FIG. 6

— VIDEO SIGNAL
— CONTROL SIGNAL
····· OTHER SIGNALS

# FIG. 7

START

INPUT SIGNAL, HEADER INFORMATION, AND ID NUMBER — Step1

INTERPOLATION PROCESS — Step2

YC CONVERSION PROCESS — Step3

WHITE BALANCE PROCESS — Step4

Step5
IS ID NUMBER 1?    NO

YES    Step6
LINEAR MATRIX CONVERSION PROCESS

Step7
TABLE CONVERSION PROCESS

SIGNAL PROCESSING — Step8

COMPRESSION PROCESS — Step9

OUTPUT SIGNAL — Step10

END

# FIG. 8

VIDEO SIGNAL
CONTROL SIGNAL
OTHER SIGNALS

EP 2 165 642 A1

FIG. 9

VIDEO SIGNAL
CONTROL SIGNAL
OTHER SIGNALS

# FIG. 10

| MODEL NUMBER | COLOR CONVERSION PROCESSING METHOD |
|---|---|
| A | method1 |
| B | method2 |
| C | method2 |
| D | method1 |
| ⋮ | ⋮ |

FIG. 11

VIDEO SIGNAL
CONTROL SIGNAL
OTHER SIGNALS

## INTERNATIONAL SEARCH REPORT

| | International application No. |
|---|---|
| | PCT/JP2008/060394 |

A. CLASSIFICATION OF SUBJECT MATTER
*A61B1/04*(2006.01)i, *H04N5/225*(2006.01)i

According to International Patent Classification (IPC) or to both national classification and IPC

B. FIELDS SEARCHED

Minimum documentation searched (classification system followed by classification symbols)
A61B1/04, H04N5/225

Documentation searched other than minimum documentation to the extent that such documents are included in the fields searched
Jitsuyo Shinan Koho          1922-1996   Jitsuyo Shinan Toroku Koho   1996-2008
Kokai Jitsuyo Shinan Koho    1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2006-142001 A  (Fuji Photo Film Co., Ltd.), 08 June, 2006 (08.06.06), Par. Nos. [0037], [0052] to [0062], [0076] to [0077], [0107] to [0109]; Figs. 1, 2, 11, 16 & US 2006/0082647 A1    & EP 1650982 A1 | 1-10 |
| Y | JP 2001-70240 A  (Olympus Optical Co., Ltd.), 21 March, 2001 (21.03.01), Par. Nos. [0028], [0029]; Fig. 7 (Family: none) | 1-10 |
| Y | JP 63-240824 A  (Toshiba Corp.), 06 October, 1988 (06.10.88), Page 3, upper right column, lines 12 to 15 (Family: none) | 6 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 June, 2008 (26.06.08) | 08 July, 2008 (08.07.08) |

| Name and mailing address of the ISA/ Japanese Patent Office | Authorized officer |
|---|---|
| Facsimile No. | Telephone No. |

Form PCT/ISA/210 (second sheet) (April 2007)

**EP 2 165 642 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO62199190 B
- JP 2001070240 A